# EUROPÄISCHE PATENTSCHRIFT

(11) **EP 4 152 033 B1**
(45) Veröffentlichungstag und Bekanntmachung des Hinweises auf die Patenterteilung: **28.05.2025**
(21) Anmeldenummer: 21196793.0
(22) Anmeldetag: 15.09.2021
(51) Int. Cl.: G01R 33/56, G01R 33/48, G01R 33/565

(54) **SPEKTRALE SÄTTIGUNG BEI DER MAGNETRESONANZTOMOGRAPHIE**
SPECTRAL SATURATION IN MAGNETIC RESONANCE IMAGING
SATURATION SPECTRALE EN TOMOGRAPHIE PAR RÉSONANCE MAGNÉTIQUE

(43) Veröffentlichungstag der Anmeldung: 22.03.2023
(73) Patentinhaber: Siemens Healthineers AG, 91301 Forchheim (DE)
(72) Erfinder: Grodzki, David, 91058 Erlangen (DE); Liebig, Patrick, 91052 Erlangen (DE); Ritter, Dieter, 90762 Fürth (DE); Schneider, Rainer, 91054 Erlangen (DE)
(74) Vertreter: Siemens Healthineers Patent Attorneys

(56) Entgegenhaltungen:
- US-A1- 2008 238 421
- US-B2- 10 598 746
- LEE JAE-SEUNG ET AL: "Uniform saturation of a strongly coupled spin system by two-frequency irradiation", THE JOURNAL OF CHEMICAL PHYSICS, vol. 134, no. 23, 21 June 2011 (2011-06-21), US, pages 234504, XP055894208, ISSN: 0021-9606, DOI: 10.1063/1.3600758
- MIYAZAKI MITSUE ET AL: "Enhanced fat suppression technique for breast imaging : Fat Suppression Technique for Breast Imaging", JOURNAL OF MAGNETIC RESONANCE IMAGING, vol. 38, no. 4, 21 November 2012 (2012-11-21), US, pages 981 - 986, XP055891998, ISSN: 1053-1807, DOI: 10.1002/jmri.23932

## Beschreibung

Die vorliegende Erfindung betrifft ein Verfahren zum Erzeugen eines Sättigungspulses für eine spektrale Sättigung bei der Magnetresonanztomographie, wobei das Verfahren ein Vorgeben oder Ermitteln einer ersten Resonanzfrequenz eines MR-Spektrums einer ersten Substanz, ein Vorgeben oder Ermitteln einer ersten Sättigungsfrequenz in einem vorbestimmten Bereich um eine zweite Resonanzfrequenz des MR-Spektrums einer zweiten Substanz und ein Erzeugen eines Sättigungspulses, der bei der ersten Resonanzfrequenz zu keiner Sättigung der ersten Substanz führt, wobei der Sättigungspuls mindestens drei Spektralpeaks zur Sättigung der zweiten Substanz in einem vorgegebenen Bereich der ersten Sättigungsfrequenz aufweist (im vorliegenden Dokument wird ein Spektralpeak auch als Sättigungspeak bezeichnet, wenn er zur Sättigung vorgesehen ist). Darüber hinaus betrifft die vorliegende Erfindung ein Verfahren zur Bildgebung in der Magnetresonanztomographie, ein Computerprogrammprodukt, ein Computer-lesbares Medium sowie ein entsprechendes Magnetresonanzsystem.

Die Magnetresonanztomographie (MRT) ist ein bildgebendes Verfahren, das vor allem in der medizinischen Diagnostik eingesetzt wird. Für die MRT wird ein hohes sowie homogenes statisches Magnetfeld benötigt, auch als B₀-Feld bezeichnet. Bei der MRT werden die Relaxationszeiten von Kernspins als Reaktion auf einen hochfrequenten (HF) Anregungspuls beobachtet, insbesondere die sogenannte longitudinale Relaxationszeit T₁ sowie die transversale Relaxationszeit T₂. Dabei spielt die Messung von Relaxationszeiten des Wasserstoffkerns, also eines Protons, aufgrund seiner Häufigkeit im menschlichen Körper die größte Rolle.

Eine Herausforderung in der MRT ist es, einen guten Kontrast zwischen Fettgewebe und wässrigem Gewebe zu erzeugen und insbesondere störende Einflüsse von Fettsignalen in wässrigen Geweben zu minimieren. Eine Trennung der Signale von Fett- und Wassermolekülen ist aufgrund der unterschiedlichen chemischen Verschiebung beider Signale möglich. Unter chemischer Verschiebung versteht man eine Verschiebung der Resonanzfrequenz eines Kerns in Abhängigkeit von seiner elektrischen und chemischen Umgebung.

Eine Methode zur Unterdrückung von Fettsignalen aufgrund der unterschiedlichen chemischen Verschiebung von Wasserstoffkernen im Fettgewebe sowie in wässrigem Gewebe ist als spektrale Fettsättigung bekannt. In einem Magnetresonanz (MR)-Spektrum zeigen sich die Resonanzen von Wasserstoffkernen in Fett- und Wassermolekülen als getrennte Signale in Form eines Fett- und Wasser-Peaks. Es wird ein spektral selektiver HF-Anregungspuls ausgesendet, der nur die Wasserstoffkerne im Fettgewebe anregt, so dass die longitudinale Magnetisierung im Fett in eine transversale Magnetisierung konvertiert wird. Diese wird sofort durch einen Magnetfeldgradienten dephasiert, so dass das Fettgewebe durch die direkt folgende Bildgebungssequenz nicht mehr dargestellt werden kann.

Die spektrale Fettsättigung ist jedoch anfällig gegenüber Inhomogenitäten des B₀-Feldes. Diese bestimmen nämlich die Position und die Breite des Fett-Peaks im MR-Spektrum. Diese Abhängigkeit bedingt somit, dass die Qualität der Fettsignalunterdrückung räumlich inhomogen verteilt sein kann. Eine Möglichkeit, die Homogenität des B₀-Feldes zu justieren, nennt man "Shimmen". Es gibt daher bei den meisten MRT-Systemen sogenannte Shim-Spulen, die in der Lage sind, auch komplexere räumliche Verläufe von Magnetfeldern zu korrigieren.

Aus der Druckschrift "Broadband Slab Selection with B1+ Mitigation at 7T via Parallel Spectral-Spatial Excitation" (Setsompop et al., MRM 2009) sind Multikanal-Anwendungen bekannt. Dort werden für pTX-Schicht- oder Slab-Anregungen (paralleler Sendebetrieb) über eine Breite von Anregungsfrequenzen durchgeführt.

Aus der Druckschrift DE 10 2012 214 660 B4 ist eine automatisierte spektrale Fettsättigung bekannt. Das Verfahren basiert auf der Aufnahme eines MR-Spektrums mit Hilfe einer HF-Spule sowie eines MR-Geräts, wobei im Aufnahmevolumen des MRT-Geräts ein statisches Magnetfeld B₀ herrscht, sowie auf einer automatischen Analyse dieses MR-Spektrums durch eine Suche nach zwei Resonanzsignalen in Form eines Fett-Peaks sowie eines Wasser-Peaks und nach einem Minimum zwischen den beiden Peaks. Eine solche automatische Analyse wird abhängig von einem vorgebbaren Kriterium für eine automatische Entscheidung darüber genutzt, ob eine Serie von Schritten durchgeführt wird, die eine Justage der Homogenität des statischen Magnetfelds B₀ mit Hilfe einer Shimm-Spule des MRT-Geräts sowie eine Berechnung eines HF-Pulses zur Fettsättigung abhängig von dem Ergebnis der obigen Suche umfasst.

Das Dokument LEE JAE-SEUNG ET AL: "Uniform saturation of a strongly coupled spin system by two-frequency irradiation", THE JOURNAL OF CHEMICAL PHYSICS, Bd. 134, Nr. 23, 21. Juni 2011 (2011-06-21), Seite 234504, XP055894208, US ISSN: 0021-9606, DOI: 10.1063/1.3600758 beschreibt eine Theorie für eine zwei-Frequenz-Sättigung. Die Parameter, die die Sättigung beeinflussen, werden diskutiert und an einem Modell-System mit Flüssigkristall experimentell untersucht. Es hat sich dabei gezeigt, dass eine Zwei-Frequenz-Einstrahlung extrem effizient sein kann, wenn die verwendeten Anregungsfrequenzen auf unterschiedlichen Seiten der charakteristischen Frequenz des Spin-Systems liegen. Der Frequenzabstand der Anregungsfrequenzen in einem zwei-Frequenz-Anregungssystem kann in einem breiten Frequenzbereich variiert werden.

Aus dem Dokument US 2008/238421 A1 ist ein Magnetresonanztomograph bekannt, der eine Einheit zum Einstellen von Abbildungsparametern und eine Bilddatenerfassungseinheit aufweist. Die Einheit zum Einstellen der Abbildungsparameter legt einen Abbildungsparameter durch Anwendung von ersten und zweiten Unterdrückungspulsen fest, wobei sich diese zumindest in Art, Mittenfrequenz oder Frequenzband unterscheiden. Die ersten und zweiten Unterdrückungspulse unterdrücken frequenzselektiv zumindest eines aus der Gruppe von Fett und Silikon. Die Bilddatenerfassungseinheit erfasst Bilddaten gemäß der gesetzten Abbildungsparameter.

Das Dokument US 10598746 B2 beschreibt einen SPAIR-Puls zur Reduktion von Artefakten bei dem Erfassen von Magnetresonanzdaten mittels einer Magnetresonanzvorrichtung. Der SPAIR-Puls, der auf Spins in einem ersten, vorbestimmten Frequenzbereich einwirkt, und ein Sättigungspuls, der auf Spins in einem zweiten vorbestimmten Frequenzbereich einwirkt, werden abgestrahlt. Ein Gradientenfeld zur räumlichen Kodierung wird gleichzeitig angelegt, sodass der Sättigungspuls auf einen Randbereich benachbart zu dem Volumensegment einwirkt. Der Randbereich begrenzt ein ellipsoides nutzbares Volumen der Magnetresonanzvorrichtung, in dem die Stärke des B0-Feldes um weniger als 30 ppm variiert. Spoiler-Gradientenfelder werden angelegt, um eine transversale Magnetisierung zu zerstören, bevor ein Hochfrequenz-Anregungspuls, angepasst an den SPAIR-Puls, ausgesendet wird. Magnetresonanzdaten werden nach dem SPAIR-Puls, dem Sättigungspuls und dem Anregungspuls erfasst. Der zweite Frequenzbereich wird an den ersten Frequenzbereich angepasst.

Die Aufgabe der vorliegenden Erfindung besteht darin, den Aufwand zur Berechnung eines Sättigungspulses zu reduzieren. Erfindungsgemäß wird diese Aufgabe gelöst durch ein Verfahren, ein Magnetresonanzsystem, ein Computerprogrammprodukt und ein Computer-lesbares Medium entsprechend den unabhängigen Patentansprüchen. Vorteilhafte Weiterbildungen der Erfindung ergeben sich aus den abhängigen Ansprüchen.

Entsprechend der vorliegenden Erfindung wird demnach ein Verfahren zum Erzeugen eines Sättigungspulses für eine spektrale Sättigung bei der Magnetresonanztomographie bereitgestellt. Mit der spektralen Sättigung soll das Signal einer spezifischen Substanz aus einem MRT-Signal unterdrückt werden. Das Verfahren kann somit Teil einer Magnetresonanzanwendung, z.B. einer MR-Bildgebung sein. Insbesondere soll eine Substanz unterdrückt werden, die beispielsweise bei der Bildgebung oder auf sonstige Weise im MRT-Signal stört. In einem konkreten Beispiel soll Fettgewebe unterdrückt werden, weshalb dann das Verfahren zur spektralen Fettsättigung verwendet werden kann.

In einem (ersten) Schritt des Verfahrens erfolgt ein Vorgeben oder Ermitteln einer ersten Resonanzfrequenz eines MR-Spektrums einer ersten Substanz. Diese erste Substanz besitzt also in dem MR-Spektrum eine erste Resonanzfrequenz. Eine solche Resonanzfrequenz lässt sich insbesondere am Ort eines Spektralpeaks verorten. Diese Resonanzfrequenz kann von vornherein bekannt sein und damit vorgegeben werden. Alternativ kann die Resonanzfrequenz auch manuell oder automatisch ermittelt werden. In jedem Fall ist dadurch bekannt, wo die erste Substanz in dem MR-Spektrum ein Maximum hervorruft.

Ähnliches gilt in einem weiteren Schritt, in dem ein Vorgeben oder Ermitteln einer ersten Sättigungsfrequenz in einem vorbestimmten Bereich um eine zweite Resonanzfrequenz des MR-Spektrums einer zweiten Substanz erfolgt. Die zweite Substanz verursacht in dem MR-Spektrum einen zweiten Spektralpeak beziehungsweise ein zweites Maximum. Dieses Maximum befindet sich am Ort der zweiten Resonanzfrequenz. Für die zweite Substanz wird nun eine erste Sättigungsfrequenz, mit der eine Sättigung der zweiten Substanz erreicht werden soll, (manuell oder automatisch) ermittelt oder vorgegeben. Der vorbestimmte Bereich, in dem die erste Sättigungsfrequenz liegt, ergibt sich entweder aus einer vorhergehenden Messung oder z.B**.** aus Erfahrung oder dergleichen. Beispielsweise führen typische Inhomognitäten des B₀-Felds zu bekannten Verschiebungen eines Fett-Peaks. In diesem Fall könnte die erste Sättigungsfrequenz bei der Resonanzfrequenz des verschobenen Fett-Peaks liegen. Der Bereich kann sehr klein und im Grenzfall sogar null sein. Ferner kann der Bereich auch relativ zu der ersten Resonanzfrequenz definiert sein (z.B. - 3,0 bis -3,5 ppm). Der Bereich kann alleine durch seine Außengrenzen definiert sein (z.B. -3,0 ppm und -3,5 ppm).

Für die Sättigung ist es notwendig, im Rahmen des erfindungsgemäßen Verfahrens einen Sättigungspuls zu erzeugen. Dieses Erzeugen des Sättigungspulses beinhaltet nicht nur das rechnerische Festlegen der Parameter des Pulses, sondern auch das physikalische Aussenden dieses Sättigungspulses. Dieser Sättigungspuls ist derart spezifiziert, dass er bei der ersten Resonanzfrequenz zu keiner Sättigung der ersten Substanz führt. Da das MR-Spektrum selbst einer einzigen Verbindung kein Linienspektrum, sondern ein Verteilungsspektrum ist, besitzt es bei jeder Frequenz einen von null verschiedenen Wert. Diese Werte sind jedoch in der Regel bei einem gewissen Abstand von der Resonanzfrequenz vernachlässigbar. So besitzt beispielsweise auch Fett bei der Resonanzfrequenz von Wasser einen geringen Spektralanteil, der aber in der Regel vernachlässigbar ist. In der Praxis geht man davon aus, dass der Sättigungspuls bei der ersten Resonanzfrequenz zu keiner Sättigung der ersten Substanz führt. Dies bedeutet, dass die erste Substanz unmittelbar nach dem Sättigungspuls praktisch kein MR-Signal liefert.

Des Weiteren ist der Sättigungspuls derart gestaltet, dass er einen ersten Spektralpeak zur Sättigung der zweiten Substanz bei der ersten Sättigungsfrequenz besitzt. Während also die erste Substanz durch den Sättigungspuls nicht gesättigt werden kann, wird die zweite Substanz bei Anregung durch den Sättigungspuls zumindest unter gewissen Bedingungen (z. B. des B₀-Felds) gesättigt. Vorzugsweise wird die zweite Substanz mit einem Anteil von 80 bis 100 % gesättigt. Bei 100 % spricht man von vollständiger Sättigung. Eine derartige vollständige Sättigung ist in der Regel immer erstrebenswert, kann meist aber nicht erreicht werden. Somit besitzt der Sättigungspuls bei der ersten Sättigungsfrequenz ein Maximum und bei der ersten Resonanzfrequenz einen möglichst geringen Wert.

Darüber hinaus ist vorgesehen, dass der Sättigungspuls einen zweiten Spektralpeak zur Sättigung der zweiten Substanz bei einer von der ersten Sättigungsfrequenz verschiedenen zweiten Sättigungsfrequenz in dem vorbestimmten Bereich besitzt. Der Sättigungspuls besitzt also mindestens zwei Spektralpeaks zur Sättigung der zweiten Substanz (z.B. für zwei verschiedene Bedingungen in Bezug auf die Homogenität des B₀-Felds) sowie an der Stelle der ersten Resonanzfrequenz einen Spektralanteil, der zu keiner Sättigung der ersten Substanz führt. Es soll also gezielt mit zwei Spektralpeaks eine Sättigung der zweiten Substanz bei unterschiedlichen Bedingungen herbeigeführt werden können. Mit dem zweiten Spektralpeak soll also erreicht werden können, dass die zweite Substanz auch dann gesättigt wird, wenn sich beispielsweise die MR-Resonanzfrequenz der zweiten Substanz aufgrund einer Abweichung des B₀-Felds verschiebt. Dies hat zur Folge, dass die zweite Substanz sicherer gesättigt werden kann. Ist beispielsweise eine Fettsättigung gewünscht, so lässt sich mit dem zweiten Fettsättigungspeak erreichen, dass der Anteil der Fettsättigung erhöht ist und Fettgewebe beispielsweise in einem MR-Bild dadurch dunkler erscheint und andere Gewebeabschnitte, die nicht oder nur weniger auf Fettgewebe basieren, nicht verdeckt. Mit diesem zweiten Spektralpeak kann somit gezielter ein MR-Peak der zweiten Substanz deaktiviert werden.

Darüber hinaus besitzt der Sättigungspuls - wie oben bereits angedeutet wurde - neben dem ersten und zweiten Spektralpeak mindestens einen weiteren Spektralpeak zur Sättigung der zweiten Substanz. Der Sättigungspuls besitzt in diesem Fall also mindestens drei Spektralpeaks, um eine Sättigung der zweiten Substanz zu erreichen. Auf diese Weise lassen sich gezielt mehrere Spektralanteile der zweiten Substanz in dem MR-Verfahren deaktivieren. Gegebenenfalls kann auch die Breite der einzelnen Spektralpeaks verändert werden.

Es ergibt sich damit der besondere Vorteil, dass sich Schwankungen der B₀-Karte für eine Schicht eines zu untersuchenden Objekts nicht zu sehr auf die Bildgebung bei Sättigung der zweiten Substanz (z.B. Fettsättigung) auswirken. Ein zweiter, dritter, vierter etc. Spektralpeak im Sättigungspuls für die Sättigung der zweiten Substanz erhöht nämlich die Wahrscheinlichkeit, dass die zweite Substanz auch mit einem hohen Grad (vorzugsweise 100 %) gesättigt wird. Gleichzeitig ergibt sich damit aber auch der Vorteil, dass Schwankungen des B₀-Felds von Schicht zu Schicht aufgrund der mehreren Spektralpeaks zur Sättigung der zweiten Substanz weniger ins Licht fallen. Dies wiederum führt dazu, dass ein Sättigungspuls nicht nur für eine einzige Schicht, sondern gegebenenfalls auch für eine oder mehrere benachbarte Schichten verwendet werden kann.

In einer bevorzugten Ausführungsform handelt es sich bei der ersten Substanz um Wasser und bei der zweiten Substanz um Fett. Es soll also mit dem erfindungsgemäßen Verfahren die Möglichkeit geschaffen werden, eine hohe Fettsättigung zu erzielen, wohingegen Strukturen, die auf Wasserverbindungen beruhen, nicht gesättigt werden sollen. Hierdurch wird erreicht, dass insbesondere bei der MR-Bildgebung Strukturen eines Organismus, die auf Wasserverbindungen beruhen, nicht durch Fettgewebe verdeckt werden. Da das Fettgewebe in diesem Fall kein oder nur ein geringes MR-Signal liefert, erscheint es dunkel, so dass auch Gewebeanteile, die nur ein schwaches Signal liefern, sichtbar gemacht werden können.

Bei einer weiteren Ausführungsform ist vorgesehen, dass jeder Spektralpeak des Sättigungspulses mit einem Peak eines MR-Spektrums der zweiten Substanz korrespondiert. Dies ist beispielsweise dann besonders sinnvoll, wenn die zweite Substanz in dem MR-Spektrum mehrere Peaks besitzt. In diesem Fall kann die zweite Substanz in dem MR-Signal mehrere Anteile liefern, die es einzeln zu sättigen gilt. Letztlich kann somit eine umfangreichere Sättigung der zweiten Substanz erzielt werden.

Bei einer besonders vorteilhaften Ausgestaltung des Verfahrens wird vor dem Erzeugen des Sättigungspulses eine ortsaufgelöste B0-Feldkarte eines magnetischen Gleichfelds der Magnetresonanztomographie ermittelt, und abhängig von der ortsaufgelösten B0-Feldkarte wird eine spektrale Verschiebung des Sättigungspulses an jeweiligen Orten der B0-Feldkarte bewirkt. Vorzugsweise wird die B0-Feldkarte in Abhängigkeit von durch einen Patienten induzierten B0-Abweichungen ermittelt. Ferner kann die B0-Feldkarte in Abhängigkeit von durch Gradientenfelder verursachten dynamischen Effekten zum Zeitpunkt des Anregungspulses ermittelt werden. Die B0-Feldkarte weist Daten über räumliche Variationen des B0-Feldes im Aufnahmebereich des Magnetresonanztomographen auf. Als B0-Feld wird dabei im Gegensatz zum B1-Feld ein Magnetfeld angesehen, das zeitliche Variationen nur mit Frequenzen aufweist, die wesentlich unter der Larmorfrequenz liegen, beispielsweise um den Faktor 10, 50 oder größer. Die B0-Feldkarte kann in einem Speicher der Steuerung gespeichert sein, beispielsweise wenn diese bereits bei der Herstellung durch Messung mit einer Feldkamera oder durch Berechnung eine Feldverteilung für den Magnetresonanztomographen ermittelt wurde. Es ist aber auch denkbar, dass die Steuerung zum Beginn einer Sequenz durch eine Magnetresonanzmessung oder durch Simulation, z.B**.** unter Berücksichtigung der Position und anderer Eigenschaften des Patienten oder Einstellungen des Magnetresonanztomographen wie Shim-Ströme durch Shim-Spulen diese aktuell ermittelt.

Entsprechend einer vorteilhaften Weiterbildung kann ein Parameterwert der Magnetresonanztomographie festgelegt oder automatisch ermittelt werden, und in Abhängigkeit von dem Parameterwert wird automatisch eine spektrale Lage des ersten und/oder zweiten Spektralpeaks ermittelt. Ist beispielsweise an einer bestimmten Stelle einer Schicht die Höhe des B₀-Felds exakt bekannt, so kann dadurch die spektrale Lage eines Peaks genauer ermittelt werden, so dass der Sättigungspuls entsprechend genauer justiert werden kann. Der Parameterwert der Magnetresonanztomographie kann sich jedoch auch auf eine bestimmte Region des zu untersuchenden Objekts (z.B. menschlichen Körpers) beziehen. So kann beispielsweise durch den Parameterwert eine bestimmte Körperregion festgelegt werden. Beispielsweise liegt der Spektralpeak für Fett für die Kopfregion bei einer anderen Resonanzfrequenz als bei der Nackenregion. Falls nun durch den Parameterwert die entsprechende Region des Körpers beziehungsweise Objekts festgelegt wird, kann damit auch die Lage des Spektralpeaks beziehungsweise Sättigungspeaks genauer eingestellt werden. Der Parameterwert kann sich aber auch auf andere Umgebungsvariablen beziehen, die Einfluss auf das MR-Spektrum und damit auf die Sättigungspeaks haben.

Bei einer anderen vorteilhaften Weiterbildung des erfindungsgemäßen Verfahrens ist vorgesehen, dass ein mittels der Magnetresonanztomographie zu untersuchender Raum bezüglich der ersten und zweiten Substanz in zwei Bereiche geclustert wird, und der Sättigungspuls für einen der Bereiche den ersten und den zweiten Spektralpeak und für den anderen der Bereiche nur einen einzigen Spektralpeak aufweist. Beispielsweise lässt sich ein Körper in Bereiche unterteilen, die einen überwiegenden Fettanteil oder einen überwiegenden Wasseranteil besitzen. Damit lässt sich ein Wasserbereich und ein Fettbereich festlegen. Für den Wasserbereich mit nur geringem Fettanteil kann es genügen, wenn der Sättigungspuls nur einen einzelnen Spektralpeak besitzt. Ein derartiger Sättigungspuls lässt sich mit vermindertem Aufwand berechnen. Im Fettbereich hingegen, wo der Fettanteil überwiegt, kann es sinnvoll sein, den Sättigungspuls mit zwei oder mehr Spektralpeaks für die Fettsättigung auszustatten. Dementsprechend wird die Berechnung des Sättigungspulses für den Fettbereich etwas aufwändiger. Da aber nicht in beiden Bereichen mehrere Spektralpeaks für die Fettsättigung eingesetzt werden müssen, kann in Summe Rechenzeit für die Berechnung des Sättigungspulses über alle Bereiche eingespart werden.

Der erste und zweite Spektralpeak in dem einen Bereich (z.B. Fettbereich) können eine andere Resonanzfrequenz besitzen als der einzige Spektralpeak für den anderen Bereich (z.B. Wasserbereich). So kann beispielsweise der Spektralpeak zur Fettsättigung im Wasserbereich bei -3,4 ppm liegen, während die beiden Spektralpeaks zur Fettsättigung im Fettbereich bei -3,0 ppm und -3,6 ppm liegen. Mit den beiden Spektralpeaks kann somit in einem breiteren Spektralbereich eine Fettsättigung erzielt werden. Dies ist gerade für den Fettbereich wichtig, da er im MR-Bild andernfalls von den Fettsignalen dominiert werden würde.

Die vorliegende Erfindung beinhaltet auch ein Verfahren zur Bildgebung in der Magnetresonanztomographie. Dieses Verfahren umfasst ein Erzeugen eines magnetischen Gleichfelds, nämlich des B₀-Felds. Weiterhin wird ein Anregungspuls erzeugt, der einen entsprechend dem oben geschilderten Verfahren erzeugten Sättigungspuls aufweist. Im vorliegenden Fall wird also der Anregungspuls als Gesamtpuls gesehen, der mehrere Wechselanteile beinhaltet. Zum einen werden hochfrequente Anteile des Anregungspulses dazu benutzt, um aus den entsprechenden Relaxationen der Spinpräzisionen entsprechende MR-Signale zu gewinnen, und andererseits werden hochfrequente Sättigungsanteile des Anregungspulses, nämlich der Sättigungspuls, dazu verwendet, die MR-Signale einer zweiten Substanz (z.B. Fett) zu zerstreuen, so dass diese Regionen des zu untersuchenden Objekts insbesondere nicht die gesuchten Strukturen verdecken. Es erfolgt schließlich für die Bildgebung ein Gewinnen eines Magnetresonanzsignals als Antwort auf das magnetische Gleichfeld und den Anregungspuls (einschließlich Sättigungspuls) und ein Erzeugen eines Bilds aus dem Magnetresonanzsignal. Wegen der Sättigung der zweiten Substanz (z.B. Fett) verdecken Signale der zweiten Substanz nicht die MR-Signale von anderen Substanzen.

In einer speziellen Ausgestaltung des Bildgebungsverfahrens ist vorgesehen, dass für mehrere aneinander liegende Schichten eines zu untersuchenden Objekts jeweils ein Bild gewonnen wird, und für die mehreren aneinander liegenden Schichten jeweils der gleiche Sättigungspuls verwendet wird, obwohl in den Schichten unterschiedliche Geichfeldverteilungen vorliegen. Ein einziger Sättigungspuls wird also für mehrere Schichten verwendet, auch wenn in diesen Schichten unterschiedliche Geichfeldverteilungen vorliegen. Die Geichfeldverteilungen können nicht nur regional unterschiedlich sein, auch die Mittelwerte des Gleichfelds in den einzelnen Schichten können unterschiedlich sein. Durch die mehreren Sättigungspeaks in dem Sättigungspuls kann aber trotz der Unterschiede in Gleichfeld in allen Schichten eine ausreichende Sättigung der zweiten Substanz erzielt werden. Damit ist es nicht notwendig, dass für jede Schicht ein separater Sättigungspuls errechnet wird.

In einer Weiterbildung hierzu wird automatisch entschieden, dass ein Sättigungspuls für die mehreren aneinander liegenden Schichten verwendet wird, wenn eine Abweichung des Gleichfelds in den mehreren aneinander liegenden Schichten kleiner als eine vorgegebene oder eine zur spektralen Breite des Sättigungspulses proportionale Größe ist. Es kann also automatisch festgelegt werden, ob der Sättigungspuls für mehrere Schichten eingesetzt werden kann und gegebenenfalls auch für wie viele Schichten. Diese Entscheidung lässt sich von der regionalen Änderung des Gleichfelds innerhalb einer Schicht und/oder von der Änderung des Gleichfelds von Schicht zu Schicht abhängig machen. Für die Entscheidung können entsprechende Schwellwerte genutzt werden.

Darüber hinaus wird ein Computerprogrammprodukt bereitgestellt, das in einem internen Speicher eines Computers aufgerufen werden kann und ein Computerprogramm zur Durchführung der Schritte eines oben genannten Verfahrens umfasst. Es ist also zusätzlich ein Computerprogramm vorgesehen mit Programmmitteln, die direkt in eine Speichereinheit einer Steuereinheit der MR-Anlage geladen werden können, um die Schritte des oben beschriebenen oder nachfolgend beschriebenen Verfahrens auszuführen, wenn die Programmmittel in der Steuereinheit ausgeführt werden. Ebenso wird ein computerlesbares Medium, d.h. ein elektronisch lesbarer Datenträger, bereitgestellt, auf dem das Computerprogrammprodukt obiger Art ausführbar gespeichert ist. Auf dem Datenträger sind also elektronisch lesbare Steuerinformationen gespeichert. Diese sind derart ausgestaltet, dass sie bei Verwendung des Datenträgers in einer Steuereinheit der MR-Anlage das oben oder nachfolgend beschriebene Verfahren durchführen.

Die oben genannte Aufgabe wird erfindungsgemäß auch gelöst durch ein Magnetresonanzsystem aufweisend
- eine Datenverarbeitungseinrichtung zum Vorgeben oder Ermitteln einer ersten Resonanzfrequenz eines MR-Spektrums einer ersten Substanz, und zum Vorgeben oder Ermitteln einer ersten Sättigungsfrequenz in einem vorbestimmten Bereich um eine zweite Resonanzfrequenz des MR-Spektrums einer zweiten Substanz,
- eine HF-Spule zum Erzeugen eines Sättigungspulses, der bei der ersten Resonanzfrequenz im Wesentlichen zu keiner Sättigung der ersten Substanz führt, wobei
- der Sättigungspuls einen ersten Spektralpeak zur Sättigung der zweiten Substanz bei der ersten Sättigungsfrequenz besitzt, wobei
- der Sättigungspuls einen zweiten Spektralpeak zur Sättigung der zweiten Substanz bei einer von der ersten Sättigungsfrequenz verschiedenen zweiten Sättigungsfrequenz in dem vorbestimmten Bereich besitzt,
wobei die Datenverarbeitungseinrichtung ausgelegt ist, den Sättigungspuls so zu bestimmen, dass der Sättigungspuls neben dem ersten und zweiten Spektralpeak mindestens einen weiteren Spektralpeak zur Sättigung der zweiten Substanz besitzt.

Die im Zusammenhang mit dem oben geschilderten erfindungsgemäßen Verfahren aufgezeigten Vorteile und Weiterbildungen ergeben sich sinngemäß auch für das Magnetresonanzsystem. Das Magnetresonanzsystem besitzt entsprechende Mittel wie die Datenverarbeitungseinrichtung und die HF-Spule, die zum Ausführen der oben genannten Verfahrensschritte ausgebildet sind.

Die oben dargelegten Merkmale und nachfolgend beschriebenen Merkmale können nicht nur in den entsprechenden explizit dargelegten Kombinationen verwendet werden, sondern auch in weiteren Kombinationen, soweit es nicht explizit anders erwähnt ist.

Die Erfindung wird nun anhand der beigefügten Zeichnungen näher erläutert, in denen zeigen:
- FIG 1: ein MR-Spektrum mit Spektralpeaks zur Sättigung;
- FIG 2: ein schematisches Ablaufdiagramm einiger Schritte eines erfindungsgemäßen Verfahrens;
- FIG 3: ein MRT-Bild eines Rückens mit Standard-Fettsättigung;
- FIG 4: eine gemessene B0-Feldkarte;
- FIG 5: ein MRT-Bild eines Rückens mit erfindungsgemäßer Fettsättigung;
- FIG 6: ein MRT-Bild eines Schädels mit Standard-Fettsättigung;
- FIG 7: ein MRT-Bild eines Schädels mit erfindungsgemäßer Fettsättigung und
- FIG 8: den prinzipiellen Aufbau eines MRT-Systems.

Die nachfolgend näher geschilderten Ausführungsbeispiele stellen bevorzugte Ausführungsformen der vorliegenden Erfindung dar. In den Figuren bezeichnen gleiche Bezugszeichen gleiche oder ähnliche Elemente. Weiterhin sind die Figuren schematische Darstellungen verschiedener Ausführungsformen der Erfindung. Die in den Figuren dargestellten Elemente sind nicht notwendigerweise maßstabsgetreu dargestellt. Vielmehr sind diese derart wiedergegeben, dass ihre Funktion und ihr Zweck für den Fachmann verständlich werden. Die in den Figuren dargestellten Verbindungen zwischen funktionellen Einheiten oder sonstigen Elementen können auch als indirekte Verbindungen implementiert werden, wobei eine Verbindung drahtlos oder drahtgebunden erfolgen kann. Funktionelle Einheiten können als Hardware, Software oder eine Kombination aus Hard- und Software implementiert werden.

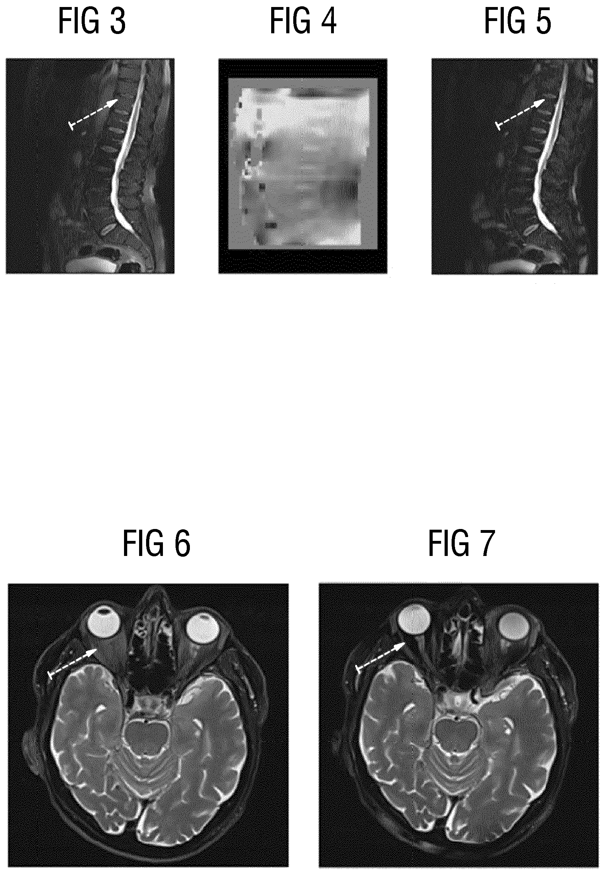

Ausgangspunkt der Erfindung ist ein Verfahren zur spektralen Sättigung von Stoffen bzw. Substanzen wie Fett oder Wasser, wobei Abweichungen des magnetischen Gleichfelds (B₀-Feld) im Bildbereich - induziert beispielsweise durch das System (Magnet, Wirbelströme) oder den Patienten (Anatomie, z.B. im Nacken) - so berücksichtigt werden, dass an jedem Ortspunkt die korrekte, gegebenenfalls bezüglich B₀ verschobene Zielfrequenz gesättigt wird. Das Verfahren beruht zum einen auf der Bestimmung der B0-Feldkarte (vergleiche FIG 4) durch Messung und/oder Simulation und zum anderen auf den dynamischen Anregungspulsen, die beispielsweise in einem sogenannten PTX-Framework (parallele Sendetechnik) berechnet werden.

Die Problematik der herkömmlichen Fettsättigung kann in den FIG 3 und 4 erkannt werden. FIG 3 zeigt ein MRT-Bild des Rückens eines Patienten. Im Mittelbereich der Wirbelsäule kann eine gute Fettsättigung erreicht werden, was sich in den dunklen Bereichen zeigt, die hellere Strukturen erkennen lassen. Hingegen im oberen Bereich des Bilds (vergleiche Pfeil) ist die Fettsättigung nicht hinreichend. Das Fettgewebe liefert MR-Signale, so dass sich graue Bereiche ergeben, welche Strukturen der Wirbelsäule verdecken. Ursache dafür sind Veränderungen des B₀-Felds beziehungsweise Inhomogenitäten, die sich systembedingt oder durch den Patienten selbst ergeben.

FIG 4 zeigt eine B₀-Feldkarte, aus der die Inhomogenitäten hervorgehen. Insbesondere im oberen linken Bereich der B0-Feldkarte zeichnen sich deutliche Abweichungen von der mittleren Magnetfeldstärke des B₀-Felds ab. Diese führen zu einer Verschiebung des Fett-Peaks im MR-Spektrum, weshalb in dem entsprechenden Bildbereich nur eine geringe Fettsättigung erzielbar ist. Bislang war also immer das Ziel, eine möglichst fehlerfreie B₀-Feldkarte als Grundlage der Berechnung des Anregungspulses beziehungsweise Sättigungspulses zu generieren.

Erfindungsgemäß wird ein alternativer Ansatz vorgeschlagen, mit dem zum einen sowohl der Einfluss von Abweichungen in der B₀-FeldkKarte verringert als auch eine vorteilhafte Weiterverwendung eines Pulses (kurz für Anregungspuls einschließlich Sättigungspuls) in benachbarten Schichten erreicht werden kann.

Bislang werden der Pulsberechnung nur zwei spektrale Randbedingungen vorgegeben, nämlich:
1. Für Wasserprotonen darf bei der Larmor-Frequenz (0 Hz Abweichung) keine Sättigung stattfinden (0 %),
2. für fettgebundene Protonen soll eine volle Sättigung (100 %) bei -3,4 ppm gegenüber der Resonanzfrequenz der Wasserprotonen (kurz: des Wassers) erfolgen.

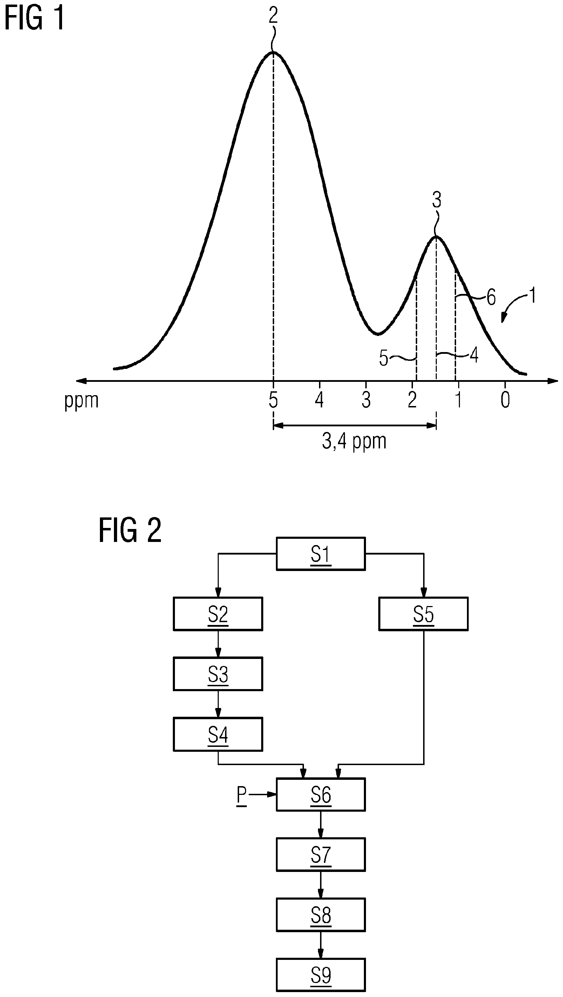

FIG 1 zeigt hierzu ein MR-Spektrum 1 von Wasser und Fett. Wasser liefert einen Spektralpeak 2 bei etwa 4,9 ppm auf der dargestellten Skala, während der Spektralpeak 3 von Fett bei ca. 1,5 ppm liegt. Beide Peaks 2 und 3 besitzen demnach einen Abstand von etwa 3,4 ppm. Diese Werte beziehen sich auf gleiche Werte des B0-Felds sowohl in dem Wasserbereich als auch in dem Fettbereich. Falls allerdings die Werte des magnetischen Gleichfelds B0 sich im Wasserbereich und im Fettbereich unterscheiden, können sich die beiden Peaks 2 und 3 zueinander verschieben. Daher kann bei homogenem Magnetfeld eine volle Fettsättigung mit einem Sättigungspuls bei - 3,4 ppm gegenüber der Resonanzfrequenz der Wasserprotonen erreicht werden, aber bei inhomogenem Magnetfeld, wenn die Resonanzfrequenz des Fettpeaks 3 nicht mehr 3,4 ppm Abstand zur Resonanzfrequenz des Wasserpeaks 2 besitzt, kann mit einem Sättigungspeak bei -3,4 ppm keine volle Sättigung erzielt werden. Ziel sollte es jedoch sein, dass mindestens 80 bis 100 % Sättigung des Fetts (allgemein der zweiten Substanz) erzielt werden. Aus diesem Grund wird die spektrale Breite des Sättigungspulses erhöht.

In FIG 1 ist für den Fall des homogenen Magnetfelds B₀ ein Fettsättigungspuls 4 mit einem Spektralpeak bei -3,4 ppm gegenüber Wasser dargestellt. Der Sättigungspeak 4 ist lediglich als Spektrallinie eingezeichnet, sie soll jedoch einen Spektralpeak repräsentieren. Um einer Verschiebung des Fettspektrums vorzubeugen, wird der Sättigungspuls dadurch verbreitert, dass ein weiterer Spektralpeak 5 beispielsweise bei -3,0 ppm (als weitere Sättigungsfrequenz) hinzugefügt wird. Der weitere Spektralpeak 5 bzw. die weitere Sättigungsfrequenz befindet sich also beispielsweise in einem vorbestimmten Bereich von 0,4 ppm um die Resonanzfrequenz des Fettpeaks 3. Wenn dann der spektrale Peak 3 von Fett aufgrund einer Inhomogenität des B₀-Felds näher an den spektralen Peak 2 von Wasser gelangt, kann durch den zweiten Sättigungspeak 5 gegebenenfalls wieder eine höhere Sättigung erzielt werden als durch den ersten Sättigungspeak 4. Es wird also für die Berechnung des Anregungs- beziehungsweise Sättigungspulses beispielsweise die folgende Bedingung hinzugefügt:
3. für fettgebundene Protonen vorzugsweise eine vollständige Sättigung (100 %) bei -3,0 ppm.

Um beispielsweise aber auch den Fall abzudecken, dass der spektrale Peak 3 von Fett weiter von dem spektralen Peak 2 des Wassers wegwandert, wird der Sättigungspuls zusätzlich mit einem dritten Sättigungspeak 6 ausgestattet werden. Dieser befindet sich beispielsweise bei -3,6 ppm (als zweite Sättigungsfrequenz) gegenüber dem Spektralpeak 2 des Wassers. Dementsprechend könnte für die Berechnung des Anregungspulses beziehungsweise Sättigungspulses folgende Zusatzbedingung eingeführt werden:
4. für fettgebundene Protonen vorzugsweise volle Sättigung (100 %) bei -3,6 ppm.

Auf diese Weise können zur Fettsättigung (allgemein: Sättigung der zweiten Substanz) einer oder mehrere Sättigungspeaks in beliebigem Abstand zu dem spektralen Peak von Wasser (allgemein: erste Substanz) in den Sättigungspuls eingefügt werden. Speziell können also auch zwischen oder außerhalb der beiden Sättigungspeaks 4, 5 zusätzliche Sättigungspeaks bei entsprechenden Sättigungsfrequenzen eingefügt werden.

Fett, welches gesättigt werden soll, hat Peaks im MR-Spektrum bei -3,3 ppm, -2,5 ppm, +0,7 ppm, -3,7 ppm, -1,8 ppm und -0,4 ppm relativ zu Wasser. Alle Peaks abzudecken, ist mit üblichen Fettsättigungsmethoden nicht möglich. Aufgrund dessen kann mit dem vorliegenden, erfindungsgemäßen Verfahren ein spektraler Fettpuls entworfen werden, welcher einen, mehrere oder alle Peaks dieses Spektrums abdecken kann.

Dies hat folgende Effekte:
- Liegen in der zur Pulsberechnung verwendeten B0-Feldkarte fehlerhafte Abweichungen vor, die jedoch kleiner als die "Breite" des Sättigungspulses sind (im Beispiel oben von -3,0 ppm bis -3,6 ppm), kann trotz der Abweichungen eine robustere Fettsättigung erreicht werden
- Liegen bei Aufnahmen mit mehreren Schichten in den Schichten unterschiedliche B0-Feldkarten vor, deren Verteilung zwischen den Schichten unterschiedlich ist, kann es nun möglich sein, dennoch einen einzigen Sättigungspuls beziehungsweise Anregungspuls für mehrere Schichten zu verwenden. Dies ist möglich, wenn sich die Abweichung des B₀-Felds an einer Position nicht weiter unterscheidet als die "Breite" des Sättigungspulses. Das vorgeschlagene Verfahren umfasst dabei auch einen Algorithmus, der anhand der gemessenen B0-Feldkarten über mehrere Schichten bestimmen kann, ob eine neue Pulsberechnung für eine Schicht benötigt wird.
- Die Pulsberechnung dauert durch die zugefügten Randbedingungen länger. In erster Näherung kann davon ausgegangen werden, dass die Rechenzeit linear mit der Anzahl der spektralen Randbedingungen ansteigt. Diese Verlängerung kann jedoch vor allem bei Multi-Schicht-Messungen dadurch ausgeglichen werden, dass insgesamt weniger oder nur ein einziger Puls berechnet werden muss.

Das Verfahren kann so ausgestaltet sein, dass die spektrale Breite des gesamten Sättigungspulses entweder durch den Benutzer vorgegeben werden kann oder automatisch anhand von weiteren Umgebungsvariablen wie B₀-Feldstärke, Körperregion etc. bestimmt werden kann. So wird beispielsweise automatisch ein zweiter Sättigungspeak 5 neben dem ersten Sättigungspeak 4 benutzt, wenn das B₀-Feld eine gewissen Inhomogenität besitzt. Falls die Inhomogenität größer ist, wird automatisch festgelegt, dass der Sättigungspuls mit einem dritten Sättigungspeak 6 ausgestattet wird. Der Einsatz der Sättigungspeaks und deren Reihenfolge kann natürlich beliebig gewählt werden.

Optional wird eine statische B0-Feldkarte des MR-Scanners zumindest des zu erfassenden Untersuchungsvolumens ermittelt. Die B0-Feldkarte kann beispielsweise in einem Speicher der Steuerung für den Magnetresonanztomographen abgelegt sein und von dort durch die Steuerung abgerufen werden. Denkbar ist aber auch ein Abrufen von einem externen Speicher oder über ein Netzwerk.

Die B0-Feldkarte kann beispielsweise bereits durch Simulation bei der Konstruktion oder durch Messung mit einer Feldkamera im Herstellungsprozess bereitgestellt werden.

Zusätzlich oder alternativ kann die Steuerung vor der Messung mittels einer vorzugsweise schnellen Sequenz eine B0-Feldkarte messen, die die durch den Patienten hervorgerufenen B0-Veränderungen zumindest im Untersuchungsvolumen zeigt. Möglich wäre es auch, die B0-Feldkarte durch Simulation, gegebenenfalls auch mit vereinfachten Annahmen, durch die Steuerung selbst bereitzustellen.

Abhängig von der ortsaufgelösten B0-Feldkarte kann nun eine spektrale Verschiebung des Sättigungspulses an jeweiligen Orten der B0-Feldkarte bewirkt werden. Dies hat die Wirkung, dass der ortsvariabel (dynamische) Sättigungspuls die Inhomogenitäten des B0-Felds ausgleicht.

In einer weiteren Ausführungsform kann das zu untersuchende Objekt beziehungsweise die B0-Feldkarte zusätzlich räumlich in Fettbereiche (z.B. Voxel > 50 % Fett) und Wasserbereiche (Voxel > 50 % Wasser) geclustert werden. Die oben beschriebenen spektralen Erweiterungen können dann auf die Fettbereiche eingeschränkt werden. D.h., eine exemplarische dritte und vierte Bedingung könnte eine Kombination aus einer spektralen Zusammensetzung aus voller Sättigung bei -3,4 ppm für Wasserbereiche und voller Sättigung bei -3,0 ppm und - 3,6 ppm für Fettbereiche sein. Dadurch werden die vorhandenen räumlich-spektralen Freiheitsgrade primär auf die problematischen Zonen konzentriert, anstatt sie für eine jeweilig räumlich globale Unterdrückung zu verbrauchen. Eine Fett-/Wasserkartierung kann von einer vorangehenden Dixon-Messung oder bevorzugt aus einer B₀-Mapping-Sequenz entnommen werden.

FIG 2 zeigt schematisch teilweise den Ablauf eines erfindungsgemäßen Verfahrens in einer Ausführungsform. Dazu erfolgt zunächst in einem optionalen Schritt S1 ein Clustern des Untersuchungsbereichs beziehungsweise der B0-Feldkarte in einem Bereich der ersten Substanz (z.B. Wasser) und in einem Bereich der zweiten Substanz (z.B. Fett). Für die Bereiche der zweiten Substanz (nachfolgend stellvertretend als Fett bezeichnet) erfolgt in einem Schritt S2 ein Vorgeben oder Ermitteln einer ersten Resonanzfrequenz des MR-Spektrums des Wassers (nachfolgend stellvertretend für die erste Substanz). In einem weiteren Schritt S3 erfolgt ein Vorgeben oder Ermitteln einer ersten Sättigungsfrequenz in einem vorbestimmten Bereich um eine zweite Resonanzfrequenz des MR-Spektrums von Fett (nachfolgend stellvertretend für die zweite Substanz). Weiterhin wird in einem Schritt S4 zur Sättigung des Fetts eine zweite Sättigungsfrequenz in dem vorbestimmten Bereich vorgegeben oder (z.B. durch Messung) ermittelt.

Abhängig von der Clusterung in Schritt S1 kann das Verfahren anstelle des Springens zu Schritt S2 zu Schritt S5 springen. In Schritt S5 wird lediglich eine einzige Sättigungsfrequenz bzw. ein einziger Sättigungspeak vorgegeben oder ermittelt. Beispielsweise reicht es in einem Wasserbereich des zu untersuchenden Objekts, nur einen einzigen Fettpeak zur Fettsättigung zu verwenden.

In einem Schritt S6, der auf Schritt S4 oder S5 folgt, wird ein Sättigungspuls, der Teil eines Anregungspulses sein kann, erzeugt. Dieser Sättigungspuls führt bei der ersten Resonanzfrequenz des Wasser zu keiner Sättigung des Wassers. Darüber hinaus besitzt der Sättigungspuls einen ersten Spektralpeak (erster Sättigungspeak bei einer ersten Sättigungsfrequenz) zur Sättigung des Fetts bei der ersten Sättigungsfrequenz, z.B. einer Resonanzfrequenz des Fetts. Schließlich besitzt der Sättigungspuls auch einen zweiten Spektralpeak (zweiter Sättigungspeak bei einer zweiten Sättigungsfrequenz) zur Sättigung des Fetts bei der von der ersten Sättigungsfrequenz verschiedenen zweiten Sättigungsfrequenz in dem vorbestimmten Bereich. Unter Umständen werden zur Erzeugung des Sättigungspulses im Schritt S6 auch einer oder mehrere Parameter P des MRT-Systems oder aus der Umgebung (z.B. des zu untersuchenden Objekts) berücksichtigt. In einem anschließenden Schritt S7 erfolgt die Erzeugung eines Anregungspulses für die MR-Analyse. Dieser Schritt S7 kann mit dem Schritt S6 gemeinsam erfolgen.

Daraufhin erfolgt in einem Schritt S8 das Gewinnen eines Magnetresonanzsignals als Antwort auf den Anregungspuls beziehungsweise Sättigungspuls. Schließlich wird in einem Schritt S9 aus dem Magnetresonanzsignal ein Bild erzeugt. Für die vorliegende Erfindung sind jedoch nur die Schritte S2, S3 und S6 von vordergründiger Bedeutung.

Die FIG 3 bis 5 zeigen anhand einer MRT-Abbildung der Wirbelsäule den Vorteil des erfindungsgemäßen Verfahrens. Wie im Zusammenhang mit den FIG 3 und 4 oben bereits erläutert wurde, sind gewisse strukturelle Bereiche im Bereich des Pfeils von FIG 3 durch Fettgewebe verdeckt, da dort nur ein herkömmliches Fettsättigungsverfahren verwendet wurde. In dem Beispiel von FIG 5 hingegen wurde im erfindungsgemäßen Verfahren mit einem zusätzlichen Spektralpeak zur Fettsättigung gearbeitet. In diesem Fall können die strukturellen Bereiche der Wirbelsäule bei dem in FIG 5 eingezeichneten Pfeil besser erkannt werden, da eine hohe Fettsättigung erzielt wurde.

Ähnliches kann durch die Schädelaufnahmen der FIG 6 und 7 bestätigt werden. Bei der Aufnahme von FIG 6 erfolgte wiederum eine herkömmliche Fettsättigung. Bei der Aufnahme von FIG 7 hingegen erfolgte die erfindungsgemäße Fettsättigung mit mindestens einem weiteren Spektralpeak. Wie die durch Pfeile gekennzeichneten Augenhöhlen zeigen, verdeckt Fettgehalt in dem Gewebe hinter dem Auge den Sehnerv. In FIG 7 ist der Sehnerv hingegen gut erkennbar, da er nicht durch fetthaltiges Gewebe in der Augenhöhle verdeckt wird.

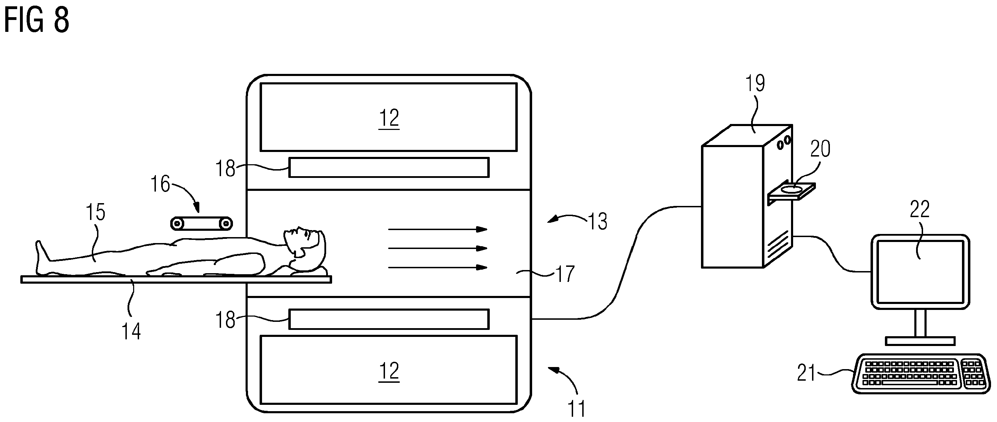

FIG 8 zeigt ein Ausführungsbeispiel eines Magnetresonanzsystems zur verbesserten spektralen Fettsättigung im Querschnitt, umfassend ein MRT-Gerät 11, eine Steuerungseinheit beziehungsweise Datenverarbeitungseinrichtung 19 und eine Eingabeeinheit 21 sowie eine Ausgabeeinheit 22. Ein computerlesbares Medium 20 (z.B. DVD, USB-Stick oder dergleichen) ist von der Datenverarbeitungseinrichtung 19 verarbeitbar. Insbesondere ist auf dem computerlesbaren Medium 20 ein Computerprogramm gespeichert, mit dem die Schritte des in FIG 2 dargestellten Verfahrens auslösbar beziehungsweise steuerbar sind. Die Datenverarbeitungseinrichtung 19 ist dementsprechend ausgelegt, um diese Verfahrensschritte zu steuern beziehungsweise durchzuführen.

Das MRT-Gerät 11 umfasst im folgenden Beispiel einen Kryostaten 12, in dem sich ein Magnet aus supraleitendem Material befindet. Typischerweise ist ein solcher Kryostat 12 mit flüssigem Helium gefüllt, um den Magneten unter die Sprungtemperatur abzukühlen und in den supraleitenden Zustand zu überführen. Ein supraleitender Magnet ist Voraussetzung, um ein hohes statisches Magnetfeld B₀ 17 bis zu einer Stärke von mehreren Tesla in einem großen Aufnahmevolumen 13 zu erzeugen. Der Kryostat 12 sowie der Magnet sind typischerweise im Wesentlichen als hohler Zylinder ausgebildet, in dessen hohlem Inneren das statische Magnetfeld B₀ 17 erzeugt werden kann. Weiterhin weist das MRT-Gerät 11 HF-Spulen 18 auf, die das Aufnahmevolumen 13 umgeben.

## Patentansprüche

1. Verfahren zum Erzeugen eines Sättigungspulses für eine spektrale Sättigung bei der Magnetresonanztomographie, wobei das Verfahren folgende Schritte aufweist:
- Vorgeben oder Ermitteln (S2) einer ersten Resonanzfrequenz eines MR-Spektrums einer ersten Substanz,
- Vorgeben oder Ermitteln (S3) einer ersten Sättigungsfrequenz in einem vorbestimmten Bereich um eine zweite Resonanzfrequenz des MR-Spektrums einer zweiten Substanz, und
- Erzeugen (S6) eines Sättigungspulses, der bei der ersten Resonanzfrequenz zu keiner Sättigung der ersten Substanz führt, wobei
- der Sättigungspuls einen ersten Spektralpeak (4) zur Sättigung der zweiten Substanz bei der ersten Sättigungsfrequenz besitzt,
wobei
- der Sättigungspuls einen zweiten Spektralpeak (5) zur Sättigung der zweiten Substanz bei einer von der ersten Sättigungsfrequenz verschiedenen zweiten Sättigungsfrequenz in dem vorbestimmten Bereich besitzt.
**dadurch gekennzeichnet, dass**
der Sättigungspuls neben dem ersten und zweiten Spektralpeak mindestens einen weiteren Spektralpeak (6) zur Sättigung der zweiten Substanz besitzt.

2. Verfahren nach Anspruch 1, wobei die erste Substanz Wasser und die zweite Substanz Fett ist.

3. Verfahren nach einem der vorhergehenden Ansprüche, wobei jeder Spektralpeak (4, 5, 6) des Sättigungspulses mit einem Peak eines MR-Spektrums der zweiten Substanz korrespondiert.

4. Verfahren nach einem der vorhergehenden Ansprüche, wobei vor dem Erzeugen des Sättigungspulses eine ortsaufgelöste B0-Feldkarte eines magnetischen Gleichfelds bzw. B₀-Feldes im Bildbereich der Magnetresonanztomographie ermittelt wird, und abhängig von der ortsaufgelösten B0-Feldkarte eine spektrale Verschiebung des Sättigungspulses an jeweiligen Orten der B0-Feldkarte bewirkt wird.

5. Verfahren nach einem der vorhergehenden Ansprüche, wobei ein Parameterwert (P) der Magnetresonanztomographie festgelegt oder automatisch ermittelt wird, und in Abhängigkeit von dem Parameterwert (P) automatisch eine spektrale Lage des zweiten Spektralpeaks (5) ermittelt wird.

6. Verfahren nach Anspruch 5, wobei der Parameterwert eine Feldstärkeschwankung eines magnetischen Gleichfelds oder eine Region eines mittels der Magnetresonanztomographie zu untersuchenden Objekts (15) betrifft.

7. Verfahren nach einem der vorhergehenden Ansprüche, wobei ein mittels der Magnetresonanztomographie zu untersuchender Raum bezüglich der ersten und zweiten Substanz in zwei Bereiche geclustert wird (S1), und der Sättigungspuls für einen der Bereiche den ersten und den zweiten Spektralpeak (4, 5) und für den anderen der Bereiche nur einen einzigen Spektralpeak aufweist.

8. Verfahren nach Anspruch 7, wobei sich der einzige Spektralpeak bezüglich seiner Resonanzfrequenz von dem ersten und dem zweiten Spektralpeak (4, 5) unterscheidet.

9. Verfahren zur Bildgebung in der Magnetresonanztomographie, wobei das Verfahren folgende Schritte aufweist:
- Erzeugen eines magnetischen Gleichfelds bzw. B₀-Feldes im Bildbereich der Magnetresonanztomographie,
- Erzeugen (S7) eines Sättigungspulses gemäß dem Verfahren nach Anspruch 1 zur Sättigung der zweiten Substanz und Erzeugen eines Anregungspulses zur Anregung der ersten Substanz,
- Gewinnen (S8) eines Magnetresonanzsignals als Antwort auf das magnetische Gleichfeld und den Anregungspuls, und
- Erzeugen (S9) eines Bildes aus dem Magnetresonanzsignal.

10. Verfahren nach Anspruch 9, wobei für mehrere aneinander liegende Schichten eines zu untersuchenden Objekts (15) jeweils ein Bild gewonnen wird, und für die mehreren aneinander liegenden Schichten jeweils der gleiche Sättigungspuls verwendet wird, obwohl in den Schichten unterschiedliche Gleichfeldverteilungen vorliegen.

11. Verfahren nach Anspruch 9 oder 10, wobei automatisch dafür entschieden wird, dass ein Sättigungspuls für die mehreren aneinander liegenden Schichten verwendet wird, wenn eine Abweichung des Gleichfelds in den mehreren aneinander liegenden Schichten kleiner als eine vorgegebene oder eine zur spektralen Breite des Sättigungspulses proportionale Größe ist.

12. Computerprogrammprodukt, umfassend Befehle, die bei der Ausführung des Programms durch einen Prozessor eines Magnetresonanzsystems bewirken, dass das Magnetresonanzsystem die Verfahrensschritte nach einem der Ansprüche 1 bis 11 ausführt.

13. Computerlesbares Medium (20), auf dem das Computerprogrammprodukt nach Anspruch 12 ausführbar gespeichert ist.

14. Magnetresonanzsystem aufweisend
- eine Datenverarbeitungseinrichtung (19) zum Vorgeben oder Ermitteln (S2) einer ersten Resonanzfrequenz eines MR-Spektrums einer ersten Substanz, und zum Vorgeben oder Ermitteln (S3) einer ersten Sättigungsfrequenz in einem vorbestimmten Bereich um eine zweite Resonanzfrequenz des MR-Spektrums einer zweiten Substanz,
- eine HF-Spule (18) zum Erzeugen eines Sättigungspulses, der bei der ersten Resonanzfrequenz zu keiner Sättigung der ersten Substanz führt, wobei
- der Sättigungspuls einen ersten Spektralpeak (4) zur Sättigung der zweiten Substanz bei der ersten Sättigungsfrequenz besitzt,
- der Sättigungspuls einen zweiten Spektralpeak (5) zur Sättigung der zweiten Substanz bei einer von der ersten Sättigungsfrequenz verschiedenen zweiten Sättigungsfrequenz in dem vorbestimmten Bereich besitzt
**dadurch gekennzeichnet, dass**
die Datenverarbeitungseinrichtung (19) ausgelegt ist, den Sättigungspuls so zu bestimmen, dass der Sättigungspuls neben dem ersten und zweiten Spektralpeak mindestens einen weiteren Spektralpeak (6) zur Sättigung der zweiten Substanz besitzt.

## Claims

1. Method for generating a saturation pulse for spectral saturation in magnetic resonance tomography, wherein the method has steps as follows:
- specifying or ascertaining (S2) a first resonance frequency of an MR spectrum of a first substance,
- specifying or ascertaining (S3) a first saturation frequency in a predefined range around a second resonance frequency of the MR spectrum of a second substance, and
- generating (S6) a saturation pulse which causes no saturation of the first substance at the first resonance frequency, wherein
- the saturation pulse has a first spectral peak (4) for saturation of the second substance at the first saturation frequency,
wherein
- the saturation pulse has a second spectral peak (5) for saturation of the second substance at a second saturation frequency, which second saturation frequency differs from the first saturation frequency, in the predefined range,
**characterised in that**
the saturation pulse has, in addition to the first and second spectral peak, at least one further spectral peak (6) for saturation of the second substance.

2. Method according to claim 1, wherein the first substance is water and the second substance is fat.

3. Method according to one of the preceding claims, wherein each spectral peak (4, 5, 6) of the saturation pulse corresponds to a peak of an MR spectrum of the second substance.

4. Method according to one of the preceding claims, wherein before the generation of the saturation pulse, a spatially resolved B₀ field map of a magnetic static field or B₀ field in the image region of the magnetic resonance tomography is ascertained and, as a function of the spatially resolved B₀ field map, a spectral shift of the saturation pulse is effected at respective locations of the B₀ field map.

5. Method according to one of the preceding claims, wherein a parameter value (P) of the magnetic resonance tomography is determined or automatically ascertained and, as a function of the parameter value (P), a spectral location of the second spectral peak (5) is automatically ascertained.

6. Method according to claim 5, wherein the parameter value relates to a fluctuation in the field strength of a magnetic static field or a region of an object (15) that is to be examined by means of the magnetic resonance tomography.

7. Method according to one of the preceding claims, wherein a volume that is to be examined by means of the magnetic resonance tomography is clustered (S1) into two regions in relation to the first and second substance, and the saturation pulse has the first and the second spectral peak (4, 5) for one of the regions and only a single spectral peak for the other region.

8. Method according to claim 7, wherein the single spectral peak differs from the first and the second spectral peak (4, 5) in respect of its resonance frequency.

9. Method for imaging in magnetic resonance tomography, wherein the method has steps as follows:
- generating a magnetic static field or B₀ field in the image region of the magnetic resonance tomography,
- generating (S7) a saturation pulse according to the method according claim 1 for saturation of the second substance and generation of an excitation pulse for excitation of the first substance,
- capturing (S8) a magnetic resonance signal as a response to the magnetic static field and the excitation pulse, and
- generating (S9) an image from the magnetic resonance signal.

10. Method according to claim 9, wherein an image is captured for each of a plurality of adjacent slices of an object (15) that is to be examined, and the same saturation pulse is used in each case for the plurality of adjacent slices, even though different static field distributions are present in the slices.

11. Method according to claim 9 or 10, wherein it is automatically decided that a saturation pulse will be used for the plurality of adjacent slices if a variation of the static field in the plurality of adjacent slices is less than a specified amount or less than an amount which is proportional to the spectral width of the saturation pulse.

12. Computer program product, comprising commands which, when the program is executed by a processor of a magnetic resonance system, cause the magnetic resonance system to carry out the method steps according to one of the claims 1 to 11.

13. Computer-readable medium (20) on which the computer program product according to claim 12 is executably stored.

14. Magnetic resonance system having
- a data processing facility (19) for specifying or ascertaining (S2) a first resonance frequency of an MR spectrum of a first substance, and for specifying or ascertaining (S3) a first saturation frequency in a predefined range around a second resonance frequency of the MR spectrum of a second substance,
- an RF coil (18) for generating a saturation pulse which causes no saturation of the first substance at the first resonance frequency, wherein
- the saturation pulse has a first spectral peak (4) for saturation of the second substance at the first saturation frequency,
- the saturation pulse has a second spectral peak (5) for saturation of the second substance at a second saturation frequency, which differs from the first saturation frequency, in the predefined range,
**characterised in that**
the data processing facility (19) is configured to determine the saturation pulse such that, in addition to the first and second spectral peak, the saturation pulse has at least one further spectral peak (6) for saturation of the second substance.

## Revendications

1. Procédé de production d'une impulsion de saturation pour une saturation spectrale dans la tomodensitométrie par résonance magnétique, dans lequel le procédé comporte les stades suivants :
- prescription ou détermination (S2) d'une première fréquence de résonance d'un spectre RM d'une première substance,
- prescription ou détermination (S3) d'une première fréquence de saturation dans une première plage déterminée à l'avance autour d'une deuxième fréquence de résonance du spectre RM d'une deuxième substance, et
- production (S6) d'une impulsion de saturation qui, à la première fréquence de résonance, ne donne pas de saturation de la première substance, dans lequel
- l'impulsion de saturation possède un premier pic (4) spectral de saturation de la deuxième substance à la première fréquence de saturation,
dans lequel
- l'impulsion de saturation possède, dans la plage déterminée à l'avance, un deuxième pic (5) spectral de saturation de la deuxième substance à une deuxième fréquence de saturation différente de la première fréquence de saturation,
**caractérisé en ce que**
l'impulsion de saturation possède outre le premier et le deuxième pics spectraux au moins un autre pic (6) spectral de saturation de la deuxième substance.

2. Procédé suivant la revendication 1, dans lequel la première substance est de l'eau et la deuxième substance est de la matière grasse.

3. Procédé suivant l'une des revendications précédentes, dans lequel chaque pic (4, 5, 6) spectral de l'impulsion de saturation correspond à un pic d'un spectre RM de la deuxième substance.

4. Procédé suivant l'une des revendications précédentes, dans lequel avant la production de l'impulsion de saturation, on détermine une carte de champ B0 à résolution dans l'espace d'un champ magnétique continu ou d'un champ B₀ dans le domaine d'image de la tomodensitométrie par résonance magnétique et, en fonction de la carte de champ B0 à résolution dans l'espace, on provoque un décalage spectral de l'impulsion de saturation aux emplacements respectifs de la carte de champ B0.

5. Procédé suivant l'une des revendications précédentes, dans lequel on fixe ou on détermine automatiquement une valeur (P) de paramètre de la tomodensitométrie par résonance magnétique et, en fonction de la valeur (P) de paramètre, on détermine automatiquement une position spectrale du deuxième pic (5) spectral.

6. Procédé suivant la revendication 5, dans lequel la valeur de paramètre concerne une fluctuation de l'intensité d'un champ magnétique continu ou une région d'un objet (15) à étudier au moyen de la tomodensitométrie par résonance magnétique.

7. Procédé suivant l'une des revendications précédentes, dans lequel on regroupe (S1) en deux parties un espace, concernant la première et la deuxième substances, à étudier au moyen de la tomodensitométrie par résonance magnétique et l'impulsion de saturation pour l'une des deux parties a le premier et le deuxième pics (4, 5) spectraux et pour l'autre des parties n'a qu'un seul pic spectral.

8. Procédé suivant la revendication 7, dans lequel le seul pic spectral se distingue en ce qui concerne sa fréquence de résonance du premier et du deuxième pics (4, 5) spectraux.

9. Procédé d'imagerie dans la tomodensitométrie par résonance magnétique, dans lequel le procédé a les stades suivants :
- production d'un champ magnétique continu ou d'un champ B₀ dans le domaine d'image de la tomodensitométrie par résonance magnétique,
- production (S7) d'une impulsion de saturation suivant le procédé de la revendication 1 pour la saturation de la deuxième substance et production d'une impulsion d'excitation pour l'excitation de la première substance,
- obtention (S8) d'un signal de résonance magnétique en réponse au champ magnétique continu et à l'impulsion d'excitation, et
- production (S9) d'une image à partir du signal de résonance magnétique.

10. Procédé suivant la revendication 9, dans lequel, pour plusieurs couches les unes à côté des autres d'un objet (15) à examiner, on obtient respectivement une image et, pour les plusieurs couches les unes à côté des autres, on utilise respectivement la même impulsion de saturation, bien qu'il y ait des répartitions de champ continu différentes dans les couches.

11. Procédé suivant la revendication 9 ou 10, dans lequel on décide automatiquement du point de savoir, si on utilise une impulsion de saturation pour les plusieurs couches les unes à côté des autres, si un écart du champ continu dans les plusieurs couches les unes à côté des autres est plus petit qu'une grandeur donnée à l'avance ou proportionnelle à la largeur spectrale de l'impulsion de saturation.

12. Produit de programme d'ordinateur, comprenant des instructions qui, lors de l'exécution du programme par un processeur d'un système de résonance magnétique, font que le système de résonance magnétique exécute les stades du procédé suivant l'une des revendications 1 à 11.

13. Support (20) déchiffrable par ordinateur, sur lequel est mis en mémoire de manière réalisable le produit de programme d'ordinateur suivant la revendication 12.

14. Système de résonance magnétique comportant
- un dispositif (19) de traitement de données pour la prescription ou la détermination (S2) d'une première fréquence de résonance d'un spectre RM d'une première substance, et pour la prescription ou la détermination (S3) d'une première fréquence de saturation dans une plage déterminée à l'avance autour d'une deuxième fréquence de résonance du spectre RM d'une deuxième substance,
- une bobine (18) HF de production d'une impulsion de saturation, qui ne donne pas, à la première fréquence de résonance, de saturation de la première substance, dans lequel
- l'impulsion de saturation possède un premier pic (4) spectral de saturation de la deuxième substance à la première fréquence de saturation,
- l'impulsion de saturation possède, dans la plage déterminée à l'avance, un deuxième pic (5) spectral de saturation de la deuxième substance à une deuxième fréquence de saturation différente de la première fréquence de saturation,
**caractérisé en ce que**
le dispositif (19) de traitement de données est conçu pour déterminer l'impulsion de saturation, de manière à ce que l'impulsion de saturation possède, outre le premier et le deuxième pics spectraux, au moins un autre pic (6) spectral de saturation de la deuxième substance.
